# EUROPÄISCHE PATENTSCHRIFT

(11) **EP 1 044 968 B1**
(45) Veröffentlichungstag und Bekanntmachung des Hinweises auf die Patenterteilung: **18.04.2007**
(21) Anmeldenummer: 99121846.2
(22) Anmeldetag: 04.11.1999
(51) Int. Cl.: C07D 239/545, C07D 239/56, C07D 498/04, C08K 5/3462, C08K 5/353, C07D 263/00, C07D 239/00

(54) **6-Amino-2-oxo-pyrimidin-4-on Derivate, deren Herstellung und deren Verwendung als Stabilisatoren von halogenhaltigen Polymeren**
6-Amino-2-oxo-pyrimidin-4-one derivatives, their preparation and their use as stabilisers for halogen-containing polymers
Dérivés de 6-amino-2-oxo-pyrimidin-4-one , leur préparation et leur utilisation comme stabilisateurs pour polymères halogénés

(30) Priorität: 06.04.1999 DE 19915388
(43) Veröffentlichungstag der Anmeldung: 18.10.2000
(73) Patentinhaber: Chemtura Vinyl Additives GmbH, 68623 Lampertheim (DE)
(72) Erfinder: Wehner, Wolfgang, Dr., 64673 Zwingenberg (DE); Friedrich, Hans-Helmut, 64686 Lautertal (DE)
(74) Vertreter: Wibbelmann, Jobst

(56) Entgegenhaltungen:
- EP-A- 0 041 479
- US-A- 2 596 041
- US-A- 3 162 635
- US-A- 4 507 477
- PECORARI P. ET AL.: "Synthesis and biological activity of pyrimido[2,1-b][1,3]thiazine, [1,3]thiazino[3,2-a]purine and [1,2,3]triazolo[4,5-d][1,3]thiazino[3,2-a] pyrimidine derivatives and thiazole analogues" IL FARMACO, IT, SOCIETA CHIMICA ITALIANA, PAVIA, Bd. 46, Nr. 7,8, Juli 1991 (1991-07) - August 1991 (1991-08), Seiten 899-911, XP000915977

## Beschreibung

Die Erfindung betrifft die Verwendung von 4-Aminopyrimidindionen der unten dargestellten Formel I zum Stabilisieren von halogenhaltigen Polymeren und Zusammensetzungen bestehend aus Verbindungen der Formel I und chlorhaltigen Polymeren, insbesondere PVC.

PVC kann durch eine Reihe von Zusatzstoffen stabilisiert werden. Verbindungen des Bleis, Bariums und Cadmiums sind dafür besonders gut geeignet, sind jedoch heute aus ökologischen Gründen oder wegen ihres Schwermetallgehalts umstritten (vgl., Seiten 303-311, und "Kunststoff Handbuch PVC", Band 2/1, W. Becker/D. Braun, Carl Hanser Verlag, 2. Aufl., 1985, Seiten 531 - 538; sowie Kirk-Othmer: "Encyclopedia of Chemical Technology", 4^{th} Ed., 1994, Vol. 12, Heat Stabilizers, S. 1071 - 1091). Man sucht daher weiter nach wirksamen Stabilisatoren und Stabilisatorkombinationen, welche frei von Blei, Barium und Cadmium sind.

1,3-disubstituierte Aminopyrimidindione sind bereits in US 3,436,362, US 4,656,209, US 4,352,903 und EP-A-0 768 336 beschrieben worden, und können nach bekannten Methoden in einem (oder mehreren) Verfahrensschritt(en) hergestellt werden.

Es wurde nun gefunden, dass sich 1,3-disubstituierte 4-Aminopyrimidindione der allgemeinen Formel I besonders gut für die Stabilisierung von chlorhaltigen Polymeren, insbesondere PVC eignen. US 2,596,041 beschreibt die Synthese von 1-Alkyl-3-hydroxyalkyl-6-amino-1,2,3,4-tetrahydro-pyrimidin-2,4-dionen; US 3,162,635 erzeugt 1-Amino-3-(2-hydroxypropyl)-6-amino-1,2,3,4-tetrahydro-pyrimidin-2,4-dion als Zwischenprodukt bei der Synthese von 1,2,3,4-Tetrahydro-pteridindionen.

Ein Gegenstand der vorliegenden Erfindung sind daher Verbindungen der allgemeinen Formel I 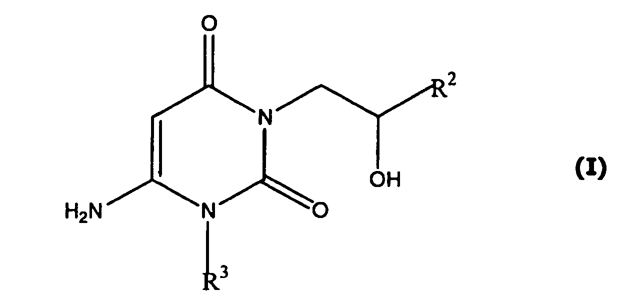 worin bedeuten
R² = H, oder die Reste C₁-C₁₄-Alkyl, C₂-C₄-Alkenyl, oder C₄-C₈-Cycloalkyl, C₆-C₁₀-Aryl, Tolyl, Xylyl, Mesityl, Propylphenyl, Butylphenyl, Trimethoxyphenyl, Dimethoxyphenyl, Propoxyphenyl und Butoxyphenyl, C₇-C₁₀-Aralkyl, -CH₂-O-R⁴, -CH₂-S-R⁴, mit R⁴ = H, C₁-C₁₀-Alkylrest, C₂-C₄-Alkenylrest oder C₄-C₈-Cycloalkyl gegebenenfalls einen Oxiranring enthaltend oder gegebenenfalls substituiert mit 1-3 C₁-C₄-Alkyl, Benzoyl- oder C₂-C₁₈-Acylrest, vorzugsweise ist R²= H, Methyl-, Ethyl-, Allyl-, Phenyl-, CH₂-O-R⁴, mit R⁴ = H, n-Propyl-, iso-Propyl-, 2-Ethyl-hexyl-, n-Butyl-, iso-Butyl-, sec-Butyl-, tert-Butyl-, Allyl-, Methallyl-, Phenyl-, Kresylreste; insbesondere ist R²= Ethyl-, CH₂-O-R⁴, mit R⁴ = H, n-Propyl-, iso-Propyl-, Allyl-,
R³ = R², R⁴ oder C₂-C₆-Alkyl mit mindestens 1 bis 5 OH- Gruppen substituiert und/oder durch mindestens 1 bis maximal 4 O-Atome unterbrochen oder C₃-C₈-Alkenyl oder - CH₂-CH(OH)R², vorzugsweise ist R³= Methyl-, Phenyl-, Benzyl-, insbesondere der Methylrest;
zum Stabilisieren von chlorhaltigen Polymeren, insbesondere Polyvinylchlorid (PVC), und deren Recyclaten.

Von diesen Verbindungen werden bevorzugt
1. Aminouracile des Typs 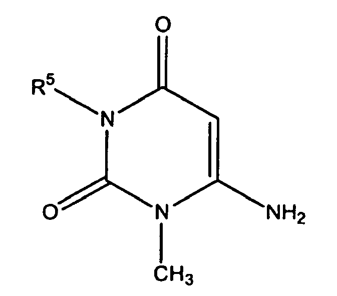
2. Aminouracile des Typs 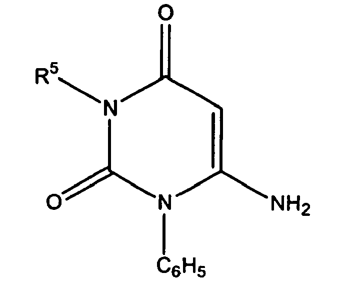
3. Aminouracile des Typs 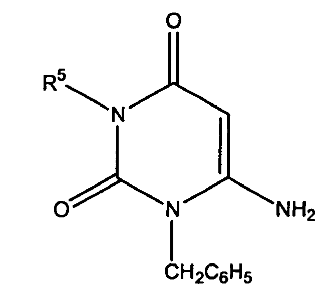
   - mit R⁵=: -CH₂CH₂OH, -CH₂-CHOH-CH₃, -CH₂-CHOH-C₂H₅,
   -CH₂-CHOH-ⁿC₃H₇, -CH₂-CHOH-ⁿC₄H₉; 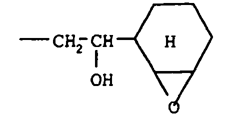
   -CH₂-CHOH-CH₂-O-ⁿC₃H₇, -CH₂-CHOH-CH₂-O-ⁱC₃H₇,
   -CH₂-CHOH-CH₂-O-ⁿC₄H₉, -CH₂-CHOH-CH₂-O-^{j}C₄H₉,
   -CH₂-CHOH-CH₂-O-^{sec}C₄H₉, -CH₂-CHOH-CH₂-O-^{t}C₄H₉,
   -CH₂-CHOH-CH₂-O-2-ethylhexyl-, -allyl-, -phenyl, -o-kresyl, -m-kresyl,
   -p-kresyl; -pentanoyl, -neohexanoyl, -neoheptanoyl, -neooctanoyl,
   -neononanoyl, -neodecanoyl.

Erfindungsgemäss besonders bevorzugt werden die Verbindungen mit
R² = Alkyl =
Pentyl, Hexyl, Heptyl, Octyl, Nonyl, Decyl, Undecyl, Dodecyl, Tridecyl und Tetradecyl, bevorzugt sind Wasserstoff, Methyl, Ethyl, n-Propyl, iso-Propyl, n-, iso-, sec- oder tert.-Butyl, und besonders bevorzugt Methyl und Ethyl;
R²= C₆-C₁₀-Aryl, Tolyl, Xylyl, Mesityl, Propylphenyl, Butylphenyl, Trimethoxyphenyl, Diethoxyphenyl, Propoxyphenyl und Butoxyphenyl,bevorzugt ist Phenyl.
R² = C₇-C₁₀-Aralkyl =
Benzyl, α- oder β-Phenylethyl, Phenylpropyl und Phenylbutyl. Bevorzugt ist Benzyl und β-Phenylethyl, besonders bevorzugt Benzyl.
R² = C₄-C₈-Cycloalkyl =
Cyclopentyl und Cyclohexyl, bevorzugt ist Cyclohexyl.
R⁴ = H, C₁-C₁₀-Alkyl, C₂-C₄-Alkenyl, Phenyl oder Benzyl, bevorzugt ist Ethyl, Propyl, Butyl, Allyl, Methallyl. Besonders bevorzugt sind Propyl, Butyl, Allyl.
R⁴ = C₂-C₁₈-Acyl =
Acetyl, Propionyl, Butyryl, Caproyl, Decanoyl, Lauroyl, Myristoyl, Palmitoyl, Stearoyl und Benzoyl; bevorzugt ist Acetyl, Propionyl, Decanoyl und Benzoyl, besonders bevorzugt ist Neodecanoyl.
R⁴ = Cycloalkyl =
Cyclohexylmethyl, Cyclohexylethyl, Cyclohexylpropyl und Cyclohexylbutyl.
R⁴ = Butenyl, Hexenyl, Heptenyl und Octenyl; bevorzugt sind Allyl und Methallyl.

Ein weiterer Gegenstand der Erfindung sind demnach Zusammensetzungen aus mindestens einer der Verbindungen der allgemeinen Formel I und chlorhaltigen Polymeren, insbesondere PVC, und deren Recyclate.

Weitere Gegenstände der Erfindung sind durch die Ansprüche gekennzeichnet.

Die erfindungsgemässen Verbindungen der Formel I sind herstellbar aus Verbindungen der allgemeinen Formel III 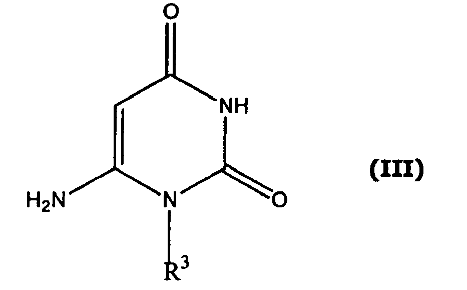 mit einer Epoxyverbindung der allgemeinen Formel V 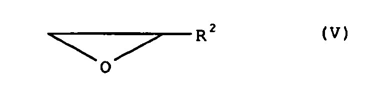 worin R² die oben angegebene Bedeutung hat, in einem Lösungsmittel, gegebenenfalls bei erhöhtem Druck, und einer katalytischen Menge Base.

Die Verbindungen der Formel III und IV werden nach bekannten Methoden hergestellt (z.B. V. Papesch u. E. F. Schroeder, J. Org. Chem., 1951 (16), 1879-1890; A. H. Nathan u. M. T. Bogert, JACS 63, 2567 (1941); W. Pfleiderer, Chem. Ber., 90, 2272 (1957) oder DRP 155 732 (1903) Farbenfabriken Bayer).

Die Verbindungen der allgemeinen Formel (V) sind aliphatische, cycloaliphatische, aromatische, araliphatische oder alkaromatische Epoxyverbindungen, worin R² die oben angegebene Bedeutung hat, wie beispielsweise Ethylenoxid, Propylenoxid, Butylenoxid, Hexylenoxid, Styroloxid, Glycidol, Glycidylether oder Glycidylthioether wie z. B. Ethyl, Propyl, Butyl, Hexyl, Octyl, Allyl und Phenylether oder Glycidylester auf Basis von aliphatischen und/oder aromatischen Alkoholen, insbesondere gegegebenenfalls verzweigten aliphatischen monofunktionellen Alkoholen wie Methanol, Ethanol, n-Propanol, iso-Propanol, Butanol, sek. Butanol, 2-Methyl-propanol-1, 2-Methyl-propanol-2, 2-Ethyl-hexanol; aromatischen Alkoholen wie insbesondere Phenol, Kresol, p-tert-Butylphenol, Thymol, Benzylalkohol.

Es handelt sich um handelsübliche Produkte, welche unter ihrer chemischen Bezeichnung oder unter dem jeweiligen Markennamen verkauft werden, wie beispielsweise EUREPOX ^{(R)} RV- K, EUREPOX ^{(R)} RV- P, EUREPOX ^{(R)} RV- A, (EUREPOX ^{(R)} = Marke der Firma Witco GmbH) oder Grilonit ^{(R)} V 51-54, Grilonit ^{(R)} RV 1802, Grilonit ^{(R)} 1804, Grilonit ^{(R)} 1805, Grilonit ^{(R)} 1807 (Grilonit ^{(R)} = Marke der Firma EMS-Chemie) oder Araldit ^{(R)} DY 0390, Araldit ^{(R)} DY 0391 (Araldit ^{(R)} = Marke der Firma CIBA Spezialitäten Chemie).

Das Verfahren zur Herstellung der erfindungsgemässen Verbindungen der allgemeinen Formel I wird erfindungsgemäss bevorzugt so durchgeführt, dass ein Gemisch aus einem Mol einer Verbindung der allgemeinen Formel III, 200-500 ml eines Lösungsmittels, 0.01-0.3 Mol einer Base und 1-2, vorzugsweise 1-1,5 Mol einer Verbindung der allgemeinen Formel V während 1-10 h bei 20-150 °C, vorzugsweise zwischen 40-90 °C, gegebenenfalls unter erhöhtem Druck, zur Reaktion gebracht wird.

Als Lösungsmittel werden vorzugsweise polare, protische und/oder aprotische Lösungsmittel wie Wasser, Methanol, Ethanol, Propanol oder sonstige mit Wasser mischbare Lösungsmittel wie Aceton, Tetrahydrofuran (THF), Dioxan, Dimethylformamid (DMF),Dimethylacetamid (DMA) oder deren Mischungen verwendet. Die Auswahl ist abhängig von der Art der Reste R², R³ in den allgemeinen Formeln III und V. Die Edukte sollten darin teilweise oder ganz löslich sein. Gegebenenfalls können die gebräuchlichen Phasentransferkatalysatoren mitverwendet werden, wie z.B. quarternäre Ammoniumsalze wie Tetrabutyl-ammoniumbromid.

Als Basen werden Alkali/Erdalkali- Hydroxide insbesondere NaOH und KOH, AlkaliAlkoholate, Alkalisalze einer organischen/anorganischen Säure wie beispielsweise Natriumacetat und tertiäre Amine wie beispielsweise Triethylamin, Tributylamin, Pyridin, Dimethylanilin und Dimethylaminopyridin verwendet sowie auch basische Anionenaustauscher.

Weitere Abwandlungen und/oder Optimierung des obigen Verfahrens kann der Fachmann routinemässig vornehmen, ohne erfinderisch tätig werden zu müssen.

Die Verbindungen der Formel I sind zur Erzielung der Stabilisierung im chlorhaltigen Polymer zweckmässig zu 0,01 bis 10, vorzugsweise zu 0,05 bis 5, insbesondere zu 0,1 bis 3 Gew.-Teilen, bezogen auf 100 Gew.-Teile PVC, zu verwenden.

Ein weiterer Gegenstand der Erfindung sind Kombinationen von Verbindungen der allgemeinen Formel I mit mindestens einem anderen üblichen Additiv bzw. Stabilisator. Bevorzugt sind Polyole und Disaccharidalkohole, Perchlorat-Verbindungen, Glycidylverbindungen, Hydrotalcite, Zeolithe (Alkali bzw. Erdalkalialumosilikate), Füllstoffe, Metallseifen, Alkali und Erdalkali-Verbindungen, Gleitmittel, Weichmacher, Phosphite, Pigmente, Epoxidierte Fettsäureester und andere Epoxidverbindungen, Antioxidantien, UV-Absorber und Lichtschutzmittel, Treibmittel.

Beispiele für solche zusätzlichen Komponenten sind nachfolgend aufgeführt und erläutert. (Vgl. "Handbook of PVC-Formulating" von E. J. Wickson, John Wiley & Sons, New York 1993).

### Polyole und Disaccharidalkohole

Als Verbindungen dieses Typs kommen beispielsweise in Betracht:

Pentaerythrit, Dipentaerythrit, Tripentaerythrit, Trimethylolethan, Bistrimethylolpropan, Polyvinylalkohol, Bistrimethylolethan, Trimethylolpropan,Zucker, Zuckeralkohole. Bevorzugt sind davon die Disaccharidalkohole.

Verwendung finden können auch Polyolsirupe, wie Sorbit-, Mannit- und Maltitsirup.

Die Polyole können in einer Menge von beispielsweise 0,01 bis 20, zweckmässig von 0,1 bis 20 und insbesondere von 0,1 bis 10 Gew.-Teilen, bezogen auf 100 Gew.-Teile PVC, angewandt werden.

### Perchlorat-Verbindungen

Beispiele sind diejenigen der Formel M(ClO₄)_{n,} wobei M für Li, Na, K, Mg, Ca, Sr, Ba, Zn, Al, La oder Ce steht. Der Index n ist entsprechend der Wertigkeit von M 1, 2 oder 3. Die Perchloratsalze können mit Alkoholen (Polyolen, Cyclodextrinen) oder Ätheralkoholen bzw. Esteralkoholen komplexiert oder gelöst sein.

Die Perchloratsalze können dabei in verschiedenen gängigen Darreichungsformen eingesetzt werden; z. B als Salz oder Lösung in Wasser oder einem organischen Solvens als solches bzw. aufgezogen auf ein Trägermaterial.

Weitere Ausführungsformen werden beschrieben in EP 0 394 547, EP 0 457 471 und WO 94/24200.

Die Perchlorate können in einer Menge von beispielsweise 0,001 bis 5, zweckmässig 0,01 bis 3, besonders bevorzugt 0,01 bis 2 Gew.-Teilen, bezogen auf 100 Gew.-Teile PVC, angewandt werden.

### Glycidylverbindungen

Sie enthalten die Glycidylgruppe 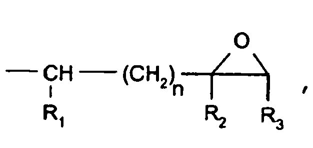 wobei diese direkt an Kohlenstoff, Sauerstoff-, Stickstoff- oder Schwefelatome gebunden ist, und worin entweder R₁ und R₃ beide Wasserstoff sind, R₂ Wasserstoff oder Methyl und n = 0 ist, oder worin R₁ und R₃ zusammen -CH₂-CH₂- oder -CH₂-CH₂-CH₂- bedeuten, R₂ dann Wasserstoff und n = 0 oder 1 ist.

Vorzugsweise finden Glycidylverbindungen mit zwei funktionellen Gruppen Verwendung. Es können aber auch prinzipiell Glycidylverbindungen mit einer, drei oder mehr funktionellen Gruppen eingesetzt werden.

Vorwiegend werden Diglycidylverbindungen mit aromatischen Gruppen eingesetzt.

Die endständigen Epoxidverbindungen können in einer Menge von vorzugsweise mindestens 0,1 Teil, beispielsweise 0,1 bis 50, zweckmässig 1 bis 30 und insbesondere 1 bis 25 Gew.-Teilen, bezogen auf 100 Gew.-Teile PVC, eingesetzt werden.

### Hydrotalcite

Die chemische Zusammensetzung dieser Verbindungen ist dem Fachmann bekannt, z.B. aus den Patentschriften DE 3 843 581, US 4,000,100, EP 0 062 813 und WO 93/20135.

Verbindungen aus der Reihe der Hydrotalcite können durch die folgende allgemeine Formel

M²⁺₁₋ₓ M³⁺ₓ(OH)₂(A^{b-})_{x/b}• d H₂O

beschrieben werden, wobei
- M²⁺: = eines oder mehrere der Metalle aus der Gruppe Mg, Ca, Sr, Zn oder Sn ist,
- M³⁺: = Al, oder B ist,
- Aⁿ: ein Anion mit der Valenz n darstellt,
b eine Zahl von 1 - 2 ist,
0 < x 0,5 ist,
- m: eine Zahl von 0 - 20 ist.

Bevorzugt sind Verbindungen mit
- Aⁿ: = OH⁻, ClO₄⁻, HCO₃⁻, CH₃COO⁻, C₆H₅COO⁻, CO₃²⁻,(CHOHCOO)₂²⁻, (CH₂COO)₂²⁻, CH₃CHOHCOO⁻, HPO₃⁻ oder HPO₄²⁻;

Beispiele für Hydrotalcite sind
Al₂O₃.6MgO.CO₂.12H₂O (i), Mg_{4,5}Al₂(OH)₁₃.CO₃.3,5H₂O (ii), 4MgO.Al₂O₃.CO₂.9H₂O (iii), 4MgO.Al₂O₃.CO₂.6H₂O- ZnO.3MgO.Al₂O₃.CO₂.8-9H₂O und ZnO.3MgO.Al₂O₃.CO₂.5-6H₂O -

Ganz besonders bevorzugt sind die Typen i, ii und iii.

### Zeolithe (Alkali bzw. Erdalkalialumosilikate)

Sie können durch die folgende allgemeine Formel M_{x/n}[(AlO₂)ₓ(SiO₂)_{y}].wH₂O beschrieben werden, worin n die Ladung des Kations M;
M ein Element der ersten oder zweiten Hauptgruppe, wie Li, Na, K, Mg, Ca, Sr oder Ba; y : x eine Zahl von 0,8 bis 15, bevorzugt von 0,8 bis 1,2; und
w eine Zahl von 0 bis 300, bevorzugt von 0,5 bis 30, ist.

Beispiele für Zeolithe sind Natriumalumosilikate der Formeln
Na₁₂Al₁₂Si₁₂O₄₈. 27 H₂O [Zeolith A], Na₆Al₆Si₆O₂₄. 2 NaX . 7,5 H₂O, X= OH, Halogen, ClO₄ [Sodalith]; Na₆Al₆Si₃₀O₇₂. 24 H₂O; Na₈Al₈Si₄₀O₉₆. 24 H₂O; Na₁₆Al₁₆Si₂₄O₈₀ . 16 H₂O; Na₁₆Al₁₆Si₃₂O₉₆. 16 H₂O; Na₅₆Al₅₆Si₁₃₆O₃₈₄. 250 H₂O [Zeolith Y], Na₈₆Al₈₆Si₁₀₆O₃₈₄. 264 H₂O [Zeolith X];
oder die durch teilweisen bzw. vollständigen Austausch der Na-Atome durch Li-, K-,
Mg-, Ca-, Sr- oder Zn-Atome darstellbaren Zeolithe wie
(Na,K)₁₀Al₁₀Si₂₂O₆₄. 20 H₂O ; Ca_{4,5}Na₃[(AlO₂)₁₂(SiO₂)₁₂]. 30 H₂O; K₉Na₃[(AlO₂)₁₂(SiO₂)₁₂]. 27 H₂O.

Ganz besonders bevorzugt sind Na-Zeolith A und Na-Zeolith P.

Die Hydrotalcite und/oder Zeolithe können in Mengen von beispielsweise 0,1 bis 20, zweckmässig 0,1 bis 10 und insbesondere 0,1 bis 5 Gew.-Teilen, bezogen auf 100 Gew.-Teile halogenhaltiges Polymer, angewandt werden.

Füllstoffe: Füllstoffe wie beispielsweise Calciumcarbonat, Dolomit, Wollastonit, Magnesiumoxid, Magnesiumhydroxid, Silikate, China-Clay, Talk, Glasfasern, Glaskugeln, Holzmehl, Glimmer, Metalloxide, oder Metallhydroxide, Russ, Graphit, Gesteinsmehl, Schwerspat, Glasfasern, Talk, Kaolin und Kreide können verwendet werden. Bevorzugt ist Kreide (HANDBOOK OF PVC FORMULATING E. J. Wickson, John Wiley & Sons, Inc., 1993, SS. 393 - 449) und Verstärkungsmittel (TASCHENBUCH der Kunststoffadditive, R. Gächter & H. Müller, Carl Hanser, 1990, S. 549 - 615).

Die Füllstoffe können in einer Menge von vorzugsweise mindestens 1 Teil, beispielsweise 5 bis 200, zweckmässig 10 bis 150 und insbesondere 15 bis 100 Gew.-Teilen, bezogen auf 100 Gew.-Teile PVC, eingesetzt werden.

Metallseifen: Metallseifen sind in der Hauptsache Metallcarboxylate bevorzugt längerkettiger Carbonsäuren. Geläufige Beispiele sind Stearate und Laurate, auch Oleate und Salze kürzerkettiger aliphatischer oder aromatischer Carbonsäuren wie Essigsäure, Propionsäure, Buttersäure, Valeriansäure, Hexansäure, Sorbinsäure; Oxalsäure, Malonsäure, Bernsteinsäure, Glutarsäure, Adipinsäure, Fumarsäure, Zitronensäure, Benzoesäure, Salicylsäure, Phthalsäuren, Hemimellithsäure, Trimellithsäure, Pyromellithsäure.

Als Metalle seien genannt: Li, Na, K, Mg, Ca, Sr, Ba, Zn, Al, La, Ce und Seltenerdenmetalle. Oft verwendet man sogenannte synergistische Mischungen wie Barium/Zink-, Magnesium/Zink- , Calcium/Zink- oder Calcium/Magnesium/Zink-Stabilisatoren. Die Metallseifen können einzeln oder in Mischungen eingesetzt werden. Eine Übersicht über gebräuchliche Metallseifen findet sich in Ullmanns Encyclopedia of Industrial Chemistry, 5^{th} Ed., Vol. A16 (1985), S. 361 ff.).

Die Metallseifen bzw. deren Mischungen können in einer Menge von beispielsweise 0,001 bis 10 Gew.-Teilen, zweckmässig 0,01 bis 8 Gew.-Teilen, besonders bevorzugt 0,05 bis 5 Gew.-Teilen, bezogen auf 100 Gew.-Teile PVC, angewandt werden.

Alkali und Erdalkali-Verbindungen: Darunter versteht man vornehmlich die Carboxylate der oben beschriebenen Säuren, aber auch entsprechende Oxide bzw. Hydroxide oder Carbonate. Es kommen auch deren Gemische mit organischen Säuren in Frage. Beispiele sind LiOH, NaOH, KOH, CaO, Ca(OH)₂, MgO, Mg(OH)₂, Sr(OH)₂, Al(OH)₃, CaCO₃ und MgCO₃ (auch basische Carbonate, wie beispielsweise Magnesia Alba und Huntit), sowie fettsaure Na- und K-Salze. Bei Erdalkali- und Zn-Carboxylaten können auch deren Addukte mit MO oder M(OH)₂ (M = Ca, Mg, Sr oder Zn), sogenannte "overbased" Verbindungen, zum Einsatz kommen. Bevorzugt werden zusätzlich zu den erfindungsgemässen Stabilisatoren Alkali-, Erdalkali- und/oder Aluminiumcarboxylate eingesetzt.

Gleitmittel: Als Gleitmittel kommen beispielsweise in Betracht: Montanwachs, Fettsäureester, PE-Wachse, Amidwachse, Chlorparaffine, Glycerinester oder Erdalkaliseifen, ferner Fettketone sowie Gleitmittel auf oder Kombinationen davon, wie in EP 0 259 783 aufgeführt. Bevorzugt ist Calciumstearat.

Weichmacher: Als organische Weichmacher kommen beispielsweise solche aus den folgenden Gruppen in Betracht:
A) Phthalsäureester: wie bevorzugt Di-2-ethylhexyl-, Di-iso-nonyl- und Di-isodecylphthalat, die auch unter den gebräuchlichen Abkürzungen DOP (Dioctylphthalat, Di-2-ethylhexyl-phthalat), DINP (Diisononylphthalat), DIDP (Diisodecylphthalat) bekannt sind.
B) Ester aliphatischer Dicarbonsäuren, insbesondere Ester von Adipin-, Azelain- und Sebazinsäure: wie bevorzugt Di-2-ethylhexyladipat und Di-iso-octyladipat.
C) Trimellithsäureester, beispielsweise Tri-2-ethylhexyltrimellithat, Tri-isodecyltrimellithat (Gemisch), Tri-iso-tridecyltrimellithat, Tri-iso-octyltrimellithat (Gemisch) sowie Tri-C₆-C₈-alkyl, Tri-C₆-C₁₀-alkyl-, Tri-C₇-C₉-alkyl- und Tri-C₉-C₁₁-alkyl-trimellithate. Gebräuchliche Abkürzungen sind TOTM (Trioctyltrimellitat, Tri-2-ethylhexyl-trimellitat), TIDTM (Triisodecyltrimellitat) und TITDTM (Trüsotridecyltrimellitat).
D) Epoxyweichmacher: In der Hauptsache sind das epoxidierte ungesättigte Fettsäuren wie z. B. epoxidiertes Sojabohnenöl.
E) Polymerweichmacher: Die gebräuchlichsten Ausgangsmaterialien für die Herstellung der Polyesterweichmacher sind: Dicarbonsäuren wie Adipin-, Phthal-, Azelain- und Sebacinsäure; Diole wie 1,2-Propandiol, 1,3-Butandiol, 1,4-Butandiol, 1,6-Hexandiol, Neopentylglycol und Diethylenglykol.
F) Phosphorsäureester: Eine Definition dieser Ester ist im vorstehend genannten "Taschenbuch der Kunststoffadditive" Kapitel 5.9.5, SS. 408 - 412, zu finden. Beispiele für solche Phosphorsäureester sind Tributylphosphat, Tri-2-ethylbutylphosphat, Tri-2-ethylhexylphosphat, Trichlorethylphosphat, 2-Ethylhexyl-di-phenylphosphat, Kresyldiphenylphosphat, Triphenylphosphat, Trikresylphosphat und Trixylenylphosphat. Bevorzugt sind Tri-2-ethylhexyl-phosphat sowie Reofos® 50 und 95 (Ciba Spezialitätenchemie).
G) Chlorierte Kohlenwasserstoffe (Paraffine)
H) Kohlenwasserstoffe
I) Monoester, z. B. Butyloleat, Phenoxyethyloleat, Tetrahydrofurfuryloleat und Alkylsulfonsäureester.
J) Glykolester, z. B. Diglykolbenzoate.

Eine Definition dieser Weichmacher und Beispiele für solche sind in "Kunststoffadditive", R. Gächter/H. Müller, Carl Hanser Verlag, 3. Aufl., 1989, Kapitel 5.9.6, Seiten 412 - 415, sowie in "PVC Technology ", W. V. Titow, 4^{th}. Ed., Elsevier Publ., 1984, Seiten 165 - 170 angegeben. Es können auch Mischungen unterschiedlicher Weichmacher verwandt werden.

Die Weichmacher können in einer Menge von beispielsweise 5 bis 20 Gew.-Teilen, zweckmässig 10 bis 20 Gew.-Teilen, bezogen auf 100 Gew.-Teile PVC, angewandt werden. Hart- bzw. Halbhart-PVC enthält bevorzugt bis zu 10 %, besonders bevorzugt bis zu 5 % oder keinen Weichmacher.

Pigmente: Geeignete Stoffe sind dem Fachmann bekannt. Beispiele für anorganische Pigmente sind TiO₂, Pigmente auf Zirkonoxidbasis, BaSO₄, Zinkoxid (Zinkweiss) und Lithopone (Zinksulfid/Bariumsulfat), Russ, Russ-Titandioxid-Mischungen, Eisenoxidpigmente, Sb₂O₃, (Ti,Ba,Sb)O₂, Cr₂O₃, Spinelle wie Cobaltblau und Cobaltgrün, Cd(S,Se), Ultramarinblau. Organische Pigmente sind z. B. Azopigmente, Phthalocyaninpigmente, Chinacridonpigmente, Perylenpigmente, Diketo-pyrrolopyrrolpigmente und Anthrachinonpigmente. Bevorzugt ist TiO₂ auch in mikronisierter Form. Eine Definition und weitere Beschreibungen finden sich im "Handbook of PVC Formulating", E. J.Wickson, John Wiley & Sons, New York, 1993.

Phosphite (Phosphorigsäuretriester): Bevorzugte organische Phosphite sind Distearylpentaerythrit-diphosphit, Trisnonylphenylphosphit und Phenyl-didecylphosphit. Als weiteres kommen Phosphorigsäurediester (mit o.g. Resten) sowie Phosphorigsäuremonoester (mit o.g. Resten) evtl. auch als Alkali-, Erdalkali-, Zink- oder Aluminiumsalz in Betracht. Diese Phosphorigsäureester können auch auf eine Alumosalz-Verbindung aufgebracht sein; siehe hierzu auch DE-A-4 031 818.

Die organischen Phosphite können in einer Menge von beispielsweise 0,01 bis 10, zweckmässig 0,05 bis 5 und insbesondere 0,1 bis 3 Gew.-Teilen, bezogen auf 100 Gew.-Teile PVC, angewandt werden.

Epoxidierte Fettsäureester und andere Epoxidverbindungen: Die erfindungsgemässe Stablisatorkombination kann zusätzlich vorzugsweise mindestens einen epoxidierten Fettsäureester enthalten. Es kommen dafür vor allem Ester von Fettsäuren aus natürlichen Quellen (Fettsäureglyceride), wie Sojaöl oder Rapsöl, in Frage. Es können aber auch synthetische Produkte zum Einsatz kommen, wie epoxidiertes Butyloleat. Ebenso verwendet werden können epoxidiertes Polybutadien und Polyisopren, gegebenenfalls auch in partiell hydroxylierter Form, oder Glycidylacrylat und Glycidylmethacrylat als Homo- bzw. Copolymer. Diese Epoxyverbindungen können auch auf eine Alumosalz-Verbindung aufgebracht sein; siehe hierzu auch DE-A-4 031 818.

### Antioxidantien:

Alkylierte Monophenole, z. B. 2,6-Di-tert-butyl-4-methylphenol, Alkylthiomethylphenole, z. B. 2,4-Di-octylthiomethyl-6-tert-butylphenol, Alkylierte Hydrochinone, z. B. 2,6-Di-tert-butyl-4-methoxyphenol, Hydroxylierte Thiodiphenylether, z. B. 2,2'-Thio-bis-(6-tert-butyl-4-methylphenol), Alkyliden-Bisphenole, z. B. 2,2'-Methylen-bis-(6-tert-butyl-4-methylphenol), Benzylverbindungen, z. B. 3,5,3',5'-Tetra-tert-butyl-4,4'-dihydroxydibenzylether, Hydroxybenzylierte Malonate, z. B. Dioctadecyl-2,2-bis-(3,5-di-tert-butyl-2-hydroxybenzyl)-malonat, Hydroxybenzyl-Aromaten, z. B. 1,3,5-Tris-(3,5-di-tert-butyl-4-hydroxybenzyl)-2,4,6-trimethylbenzol, Triazinverbindungen, z. B. 2,4-Bis-octylmercapto-6-(3,5-di-tert-butyl-4-hydroxyanilino)-1,3,5-triazin, Phosphonate und Phosphonite, z. B. Dimethyl-2,5-di-tert-butyl-4-hydroxybenzylphosphonat, Acylaminophenole, z. B. 4-Hydroxylaurinsäureanilid, Ester der beta-(3,5-Di-tert-butyl-4-hydroxyphenyl)-propionsäure, der beta-(5-tert-Butyl-4-hydroxy-3-methylphenyl)-propionsäure, der beta(3,5-Dicyclohexyl-4-hydroxyphenyl)-propionsäure, Ester der 3,5-Di-tert-butyl-4-hydroxyphenylessigsäure mit ein- oder mehrwertigen Alkoholen, Amide der beta-(3,5-Di-tert-butyl-4-hydroxyphenyl)-propionsäure, wie z. B. N,N'-Bis-(3,5-di-tert-butyl-4-hydroxyphenylpropionyl)-hexamethylendiamin, Vitamin E (Tocopherol) und Abkömmlinge.

Die Antioxidantien können in einer Menge von beispielsweise 0,01 bis 10 Gew.-Teilen, zweckmässig 0,1 bis 10 Gew.-Teilen und insbesondere 0,1 bis 5 Gew.-Teilen, bezogen auf 100 Gew.-Teile PVC, angewandt werden.

### UV-Absorber und Lichtschutzmittel:

Beispiele dafür sind: 2-(2'-Hydroxyphenyl)-benztriazole, wie z. B. 2-(2'-Hydroxy-5'-methylphenyl)-benztriazol, 2-Hydroxybenzophenone, Ester von gegebenenfalls substituierten Benzoesäuren, wie z. B. 4-tert-Butyl-phenylsalicylat, Phenylsalicylat, Acrylate, Nickelverbindungen, Oxalsäurediamide, wie z. B. 4,4'-Di-octyloxy-oxanilid, 2,2'-Di-octyloxy-5,5'-di-tert-butyl-oxanilid, 2-(2-Hydroxyphenyl)-1,3,5-triazine, wie z. B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, Sterisch gehinderte Amine, wie z. B. Bis(2,2,6,6-tetramethyl-piperidin-4-yl)-sebacat, Bis(2,2,6,6-tetramethyl-piperidin-4-yl)-succinat.

Treibmittel: Treibmittel sind z. B. organische Azo- und Hydrazoverbindungen, Tetrazole, Oxazine, Isatosäureanhydrid, sowie Soda und Natriumbicarbonat. Bevorzugt sind Azodicarbonamid und Natriumbicarbonat sowie deren Mischungen.

Definitionen und Beispiele für Schlagzähmodifikatoren und Verarbeitungshilfen, Geliermittel, Antistatika, Biocide, Metalldesaktivatoren, optische Aufheller, Flammschutzmittel, Antifogging-agents sowie Kompatibilisatoren sind beschrieben in "Kunststoffadditive", R. Gächter/H. Müller, Carl Hanser Verlag, 3. Aufl., 1989, und im "Handbook of Polyvinyl Chloride Formulating" E. J. Wilson, J. Wiley & Sons, 1993, sowie in "Plastics Additives" G. Pritchard, Chapman & Hall, London, 1st Ed., 1998.

Schlagzähmodifikatoren sind ferner ausführlich beschrieben in "Impact Modifiers for PVC", J. T. Lutz/D. L. Dunkelberger, John Wiley & Sons, 1992.

Beispiele für die zu stabilisierenden chlorhaltige Polymere sind:
Polymere des Vinylchlorides, Vinylidenchlorids, Vinylharze, enthaltend Vinylchlorideinheiten in deren Struktur, wie Copolymere des Vinylchlorids und Vinylester von aliphatischen Säuren, insbesondere Vinylacetat, Copolymere des Vinylchlorids mit Estern der Acryl- und Methycrylsäure und mit Acrylnitril, Copolymere des Vinylchlorids mit Dienverbindungen und ungesättigten Dicarbonsäuren oder deren Anhydride, wie Copolymere des Vinylchlorids mit Diethylmaleat, Diethylfumarat oder Maleinsäureanhydrid, nachchlorierte Polymere und Copolymere des Vinylchlorids, Copolymere des Vinylchlorids und Vinylidenchlorids mit ungesättigten Aldehyden, Ketonen und anderen, wie Acrolein, Crotonaldehyd, Vinylmethylketon, Vinylmethylether, Vinylisobutylether und ähnliche; Polymere des Vinylidenchlorids und Copolymere desselben mit Vinylchlorid und anderen polymerisierbaren Verbindungen; Polymere des Vinylchloracetates und Dichlordivinylethers; chlorierte Polymere des Vinylacetates, chlorierte polymerische Ester der Acrylsäure und der alpha-substituierten Acrylsäure; Polymere von chlorierten Styrolen, zum Beispiel Dichlorstyrol; Chlorkautschuke; chlorierte Polymere des Ethylens; Polymere und nachchlorierte Polymere von Chlorbutadiens und deren Copolymere mit Vinylchlorid, chlorierte Natur- und Synthesekautschuke, sowie Mischungen der genannten Polymere unter sich oder mit anderen polymerisierbaren Verbindungen. Im Rahmen dieser Erfindung sind unter PVC auch Copolymerisate mit polymerisierbaren Verbindungen wie Acrylnitril, Vinylacetat oder ABS zu verstehen, wobei es sich um Suspensions-, Masse- oder auch Homo- oder Co-Emulsionspolymerisate, welche mit den bisher verwendeten Stabilisatoren unzureichend stabilisiert werden konnten, handeln kann. Bevorzugt ist ein PVC-Homopolymer, auch in Kombination mit Polyacrylaten. Insbesondere gilt dies für Artikel, welche nach dem Luvithermverfahren hergestellt werden.

Ferner kommen auch Pfropfpolymerisate von PVC mit EVA, ABS und MBS in Betracht. Bevorzugte Substrate sind auch Mischungen der vorstehend genannten Homo-und Copolymerisate, insbesondere Vinylchlorid-Homopolymerisate, mit anderen thermoplastischen oder/und elastomeren Polymeren, insbesondere Blends mit ABS, MBS, NBR, SAN, EVA, CPE, MBAS, PMA, PMMA, EPDM und Polylactonen, insbesondere aus der Gruppe ABS, NBR, NAR, SAN und EVA. Die verwandten Abkürzungen für die Copolymerisate sind dem Fachmann geläufig und bedeuten folgendes: ABS: Acrylnitril-Butadien-Styrol; SAN: Styrol-Acrylnitril; NBR: Acrylnitril-Butadien; NAR: Acrylnitril-Acrylat; EVA: Ethylen-Vinylacetat. Es kommen insbesondere auch Styrol-Acrylnitril-Copolymerisate auf Acrylat-Basis (ASA) in Betracht. Bevorzugt als Komponente sind in diesem Zusammenhang Polymerzusammensetzungen, die als Komponenten (i) und (ii) eine Mischung aus 25-75 Gew.-% PVC und 75-25 Gew.-% der genannten Copolymerisate enthalten. Von besonderer Bedeutung sind als Komponente Zusammensetzungen, aus (i) 100 Gewichtsteilen PVC, und (ii) 0 -300 Gewichtsteilen ABS und/oder mit SAN modifiziertes ABS und 0-80 Gewichtsteilen der Copolymeren NBR, NAR und/oder EVA, insbesondere jedoch EVA.

Weiterhin kommen zur Stabilisierung im Rahmen dieser Erfindung auch insbesondere Recyclate chlorhaltiger Polymere in Frage, wobei es sich hierbei um die oben näher beschriebenen Polymere handelt, welche durch Verarbeitung, Gebrauch oder Lagerung eine Schädigung erfahren haben. Besonders bevorzugt ist PVC-Recyclat.

Diese erfindungsgemäss mitverwendbaren Verbindungen sowie die chlorhaltigen Polymeren sind dem Fachmann allgemein bekannt und werden detailliert beschrieben in "Kunstoffadditive", R. Gächter/H. Müller, Carl Hanser Verlag, 3. Aufl., 1989; in der DE 197 41 778 und in EP-A-967 245, auf welche hiermit ausdrücklich Bezug genommen wird.

Die erfindungsgemässe Stabilisierung ist besonders bei Hart-PVC-Formulierungen für transparente und nicht transparente Anwendungen von Vorteil, wie sie für Rohre, Profile und Platten üblich sind. Für transparente Anwendungen werden vorzugsweise Verbindungen der Formel (I) eingesetzt, welche Schmelzpunkte unterhalb ca. 190 °C aufweisen. Ebenso kann die Stabilisierung für halbharte und weiche Formulierungen sowie in Plastisolen verwendet werden. Die Stabilisierung kann ohne Schwermetallverbindungen (Sn-, Pb-, Cd-, Zn-Stabilisatoren) durchgeführt werden.

Zweckmässig kann die Einarbeitung der Stabilisatoren nach folgenden Methoden erfolgen: als Emulsion oder Dispersion (Eine Möglichkeit ist z. B. die Form einer pastösen Mischung. Ein Vorteil der erfindungsgemässen Kombination besteht bei dieser Darreichungsform in der Stabilität der Paste.); als Trockenmischung während des Vermischens von Zusatzkomponenten oder Polymermischungen; durch direktes Zugeben in die Verarbeitungsapparatur (z. B. Kalander, Mischer, Kneter, Extruder und dergleichen) oder als Lösung oder Schmelze bzw. als Flakes oder Pellets in staubfreier Form als One-Pack.

Das erfindungsgemäss stabilisierte PVC, das die Erfindung ebenfalls betrifft, kann auf an sich bekannte Weise hergestellt werden, wozu man unter Verwendung an sich bekannter Vorrichtungen wie der oben genannten Verarbeitungsapparaturen die erfindungsgemässe Stabilisatorkombination und gegebenenfalls weitere Zusätze mit dem PVC vermischt. Hierbei können die Stabilisatoren einzeln oder in Mischung zugegeben werden oder auch in Form sogenannter Masterbatches.

Das nach vorliegender Erfindung stabilisierte PVC kann auf bekannte Weisen in die gewünschte Form gebracht werden. Solche Verfahren sind beispielsweise Mahlen, Kalandrieren, Extrudieren, Spritzgiessen oder Spinnen, ferner Extrusions-Blasen. Das stabilisierte PVC kann auch zu Schaumstoffen verarbeitet werden.

Ein erfindungsgemäss stabilisiertes PVC eignet sich z. B. besonders für Hohlkörper (Flaschen), Verpackungsfolien (Tiefziehfolien), Blasfolien, Rohre, Schaumstoffe, Schwerprofile (Fensterrahmen), Lichtwandprofile, Bauprofile, Sidings, Fittings, Bürofolien und Apparatur-Gehäuse (Computer, Haushalteräte). Bevorzugt sind PVC-Hartschaumstoff-Formkörper und PVC-Rohre wie für Trink- oder Abwasser, Druckrohre, Gasrohre, Kabelkanal- und Kabelschutzrohre, Rohre für Industrieleitungen, Sickerrohre, Abflussrohre, Dachrinnenrohre und Drainagerohre. Näheres hierzu siehe "Kunststoffhandbuch PVC", Band 2/2, W. Becker/H. Braun, 2. Aufl., 1985, Carl Hanser Verlag, Seiten 1236 - 1277.

4-Aminopyrimidindione (6-Aminouracile) werden nach bekannten Methoden hergestellt [z. B. US Patent 2,598,936, WO 96/04280, J. Org. Chem. 16, 1879 - 1890 (1951), J. Org. Chem. 30, 656 (1965), JACS 82, 3973 (1960) und Synthesis 1996, Seite 459 ff., Berichte 99 3530 (1966) und CA 70 87727j (1969)].

Dargestellte Verbindungen 1 bis 22 sind in der Tabelle 1 zusammengefasst. Teile und Prozentangaben beziehen sich wie im übrigen Text auf das Gewicht, sofern nicht anders angegeben.

### Synthesebeispiel 1

14,1 g (0,1 Mol) 4-Amino-3-methyl-pyrimidindion-2.6, 8,9 g (0,12 Mol) Glycidol, 100 ml Wasser und 0,3 g Natriumhydroxid wurden 3 h unter Rühren bei 95 °C am Rückfluss erwärmt. Anschliessend wurde das Reaktionsgemisch abgekühlt, mit Eisessig auf pH 5 eingestellt, eine leichte Trübung abfiltriert und die Lösung am Rotavapor auf Rückstand eingeengt. Der harzartige Rückstand von 25,7 g wurde aus 100 ml ⁿPropanol umkristallisiert.
- Ausbeute:: 12,8 g = 59,5 % d. Th., weisse Kristalle vom Schmelzpunkt 198-201 °C

Die in Tabelle 1 aufgeführten Beispiele 2 und 3 wurden unter gleichen Bedingungen wie in Beispiel 1 beschrieben, synthetisiert.

### Synthesebeispiel 4

Ein Gemisch aus 28,2 g (0,2 Mol) 4-Amino-3-methyl-pyrimidindion-2.6, 90 ml Wasser, 10 ml ⁿPropanol, 0,3 g Natriumhydroxid und 34,9 g (0,3 Mol) 1,2-Epoxypropyl-isopropylether wurde 3,5 Stunden unter Rühren am Rückfluss erhitzt. Nach dem Abkühlen auf 10 °C wurde das nicht umgesetzte Edukt abgesaugt, und die Mutterlauge am Rotavapor auf Rückstand eingeengt. Der Rückstand von 55,7 g wurde mit 100 ml Methyl-iso-butylketon angeteigt, die weissen Kristalle isoliert und getrocknet.
- Ausbeute:: 28,9 g = 56,2 % d. Th. (Schmelzpunkt 175-176 °C aus ⁿPropanol)

Die in Tabelle 1 aufgeführten Beispiele 5, 6, 10 und 13 wurden entsprechend den Synthesebedingungen aus Beispiel 4 hergestellt.

### Synthesebeispiel 7

21,7 g (0,1 Mol) 4-Amino-3-benzyl-pyrimidindion-2.6, 17,5 g (0,15 Mol) 1,2-Epoxypropyl-iso-propylether, 90 ml Wasser, 10 ml ⁿPropanol und 0,4 g Natriumhydroxid wurden 2 Stunden am Rückfluss gerührt. Anschliessend wurde das Reaktionsgemisch auf 6 °C abgekühlt, die Kristalle abgesaugt, mit Wasser gewaschen und getrocknet.
- Ausbeute:: 26,0 g = 78,1 % d. Th., gelbe Kristalle Schmelzpunkt.184-186 °C (aus ⁿPropanol Schmelzpunkt. 194-195 °C)

Die in Tabelle 1 aufgeführten Beispiele 11, 12 und 14 wurden entsprechend den Synthesebedingungen aus Beispiel 7 hergestellt.

### Synthesebeispiel 8

14,1 g (0,1 Mol) 4-Amino-3-methyl-pyrimidindion-2,6, 34,2 g (0,15 Mol) Glycidylneodecanoat, 30 ml ⁿ-Propanol, 70 ml Wasser, 2 ml Triethylamin und 2 g Tetrabutylammoniumbromid wurden 5 Stunden unter Rühren am Rückfluss erhitzt. Das zweiphasige Reaktionsgemisch wurde nach dem Abkühlen mit 100 ml Dichlormethan versetzt, das nicht umgesetzte Edukt (6,7 g) abgesaugt, die organische Phase abgetrennt und auf Rückstand eingeengt. Der Rückstand von 40,7 g wurde dann mit 1200 ml Diethylether 30 Minuten gerührt, der weisse Niederschlag abgesaugt, mit Diethylether gewaschen und getrocknet.
- Ausbeute:: 11,3 g = 30,6 % d. Th. weisses kristallisiertes Pulver Schmelzpunkt 175-179 (aus Methylisobutylketon Schmelzpunkt 184-185 °C)

### Synthesebeispiel 9

Ein Gemisch aus 14,1 g (0,1 Mol) 4-Amino-3-methyl-pyrimidindion-2.6; 27,9 g (0,15 Mol) 2-Ethylhexylglycidylether, 30 ml ⁿPropanol, 70 ml Wasser und 0,4 g Natrium-hydroxid wurde 2 Stunden am Rückfluss gerührt. Anschliessend wurde das Reaktionsgemisch abgekühlt, das nicht umgesetzte Edukt mittels Filtration abgetrennt (7,5 g) und die Mutterlauge auf Rückstand eingeengt. Der Rückstand von 34,5 g wurde dann mit 50 ml Aceton/ 50 ml Diethylether gerührt, ein weisser Niederschlag abgetrennt, mit Diethylether gewaschen und getrocknet.
- Ausbeute:: 7,4 g = 22,6 % d. Th., weisse Kristalle Schmelzpunkt 159-160 °C

### Synthesebeispiel 15

423,3 g (3,0 Mol) 4-Amino-3-methyl-pyrimidindion-2.6, 500 g Wasser und 8 g Natrium-hydroxid wurden in einem 1,5 l-Druckgefäss auf 80 °C erwärmt und unter Rühren innterhalb 60 Minuten mit 198 g (4,5 Mol) Ethylenoxid bei einem Druck von 2-4 bar umgesetzt.

Das Gemisch wurde 60 Minuten bei 85-90 °C nachgerührt, abgekühlt, der weisse Niederschlag abgesaugt, mit Wasser gewaschen und bis zur Gewichtskonstanz getrocknet.
- Ausbeute:: 467,1 g = 84,1 % d. Th. weisse Kristalle, Schmelzpunkt: 280-82 °C

### Synthesebeispiel 16

In ein Reaktionsgefäss von 1,0 l Inhalt wurden 211,7 g (1,5 Mol) 4-Amino-3-methyl-pyrimidindion-2,6, 250 g Wasser und 6 g Natriumhydroxid vorgelegt und unter Rühren bei 40 °C 228,5 g (2,0 Mol) Allylglycidylether innerhalb 50 Minuten eingetropft. Anschliessend wurde das Reaktionsgemisch 5,75 Stunden bei 40 °C nachgerührt, auf ca. 5 °C abgekühlt und der kristalline Niederschlag abgesaugt. Der Filterkuchen wurde dann 2 x mit je 75 ml Aceton gewaschen und bis zur Gewichtskonstanz getrocknet.
- Ausbeute:: 321,4 g = 84,0 % d. Th. weisse Kristalle vom Schmelzpunkt.150 -152 °C

Das Produkt enthielt laut 1H NMR 0,5 Mol% 4-Amino-3-methyl-pyrimidindion-2,6

### Synthesebeispiel 17

12,7 g (0,1 Mol) 4-Amino-2,6-dihydroxypyrimidin, 100 ml Wasser, 24,6 g Allylglycidylether und 0,6 g Natriumhydroxid wurden 120 Stunden bei Raumtemperatur gerührt. Anschliessend wurde die klare gelbe Lösung mit 1,0 g Eisessig neutralisiert und am Rotationsverdampfer auf Rückstand eingeengt. Der Rückstand wurde dann in 100 ml Aceton gelöst, eine Trübung abfiltriert und das Filtrat in 500 ml Essigsäureethylester unter Rühren eingetroft. Es bildete sich ein Niederschlag, der durch Abdekantieren der überstehenden Lösung abgetrennt und mit 100 ml Aceton angeteigt wurde. Die ungelösten Anteile wurden abfiltriert und das Filtrat wieder auf Rückstand eingeengt.
Rückstand: 28,7 g = 81 % d. Th. gelbe viskose Flüssigkeit, nD30: 1,5225

Das Produkt enthielt laut 1H NMR 93,2 Gew.-% Bis-2-hydroxypropylallylether-Produkt und 6,8 Gew.-% monosubstituiertes Produkt.

### Synthesebeispiel 18

Ein Gemisch aus 25,4 g (0,2 Mol) 4-Amino-2,6-dihydroxypyrimidin, 30,3 g (0,42 Mol) 1,2-Butenoxid, 100 ml Wasser und 1,2 g Natriumhydroxid wurde 12 Tage bei ca. 20 °C gerührt. Die klare Lösung wurde dann mit 2,0 g Eisessig neutralisiert, am Rotavapor auf Rückstand eingeengt, mit 250 ml Aceton gelöst, eine Trübung abfiltriert und das Filtrat auf 150 g eingengt. Diese Lösung wurde dann in 600 ml Essigsäureethylester eingerührt.

Es trennte sich eine hochviskose Phase als Bodensatz ab. Die obere Phase wurde abdekantiert und auf Rückstand eingeengt.

Rückstand 1 enthielt 37,7 g gelbes Harz.

Der hochviskose Bodensatz wurde in 100 ml Methanol gelöst und ebenfalls auf Rückstand eingeengt.

Rückstand 2 enthielt 15,8 g gelbes Harz.

Der Rückstand 1 enthielt laut 1 H NMR 68,0 Gew.-% Bis-2-Hydroxybutyl-aminouracil und 32 Gew.-% monosubstituiertes 2-Hydroxybutyl-aminouracil.

Der Rückstand 2 enthielt laut 1H NMR 90,5 Gew.-% Bis-2-Hydroxybutyl-aminouracil und 9,5 Gew.-% monosubstituiertes Uracil.

### Synthesebeispiel 19

In ein Druckgefäss wurden 48 g (0,38 Mol) 4-Amino-2,6-dihydroxypyrimidin, 250 g Wasser und 2,0 g Natrium-hydroxid vorgelegt und bei 60 °C unter Rühren 44 g (1,0 Mol) Ethylenoxid unter Rühren innerhalb 10 Minuten eingetropft. Anschliessend wurde das Gemisch noch 30 Minuten bei 100 °C und einem Druck von 3 bar nachgerührt, abgekühlt und die klare, leicht gelbe Lösung auf Rückstand eingeengt.

Der Rückstand von 99,6 g wurde 2 x mit je 200 ml Aceton gewaschen und dann aus 200 ml n-Propanol umkristallisiert.
- Ausbeute:: 33,8 g = 41,3 % d. Th.,
weisse Kristalle vom Schmelzpunkt. 170-171 °C -

Nach dem Umkristallisieren aus ⁿPropanol/Wasser betrug der Schmelzpunkt 183 - 185 °C.

Dargestellte Verbindungen 1 bis 16 sind in der Tabelle 1 und Verbindungen 17 bis 19 sind in der Tabelle 2 zusammengefasst.

Teile und Prozentangaben beziehen sich wie im übrigen Text auf das Gewicht, sofern nicht anders angegeben.

**Tabelle 1**

| Reakt.: 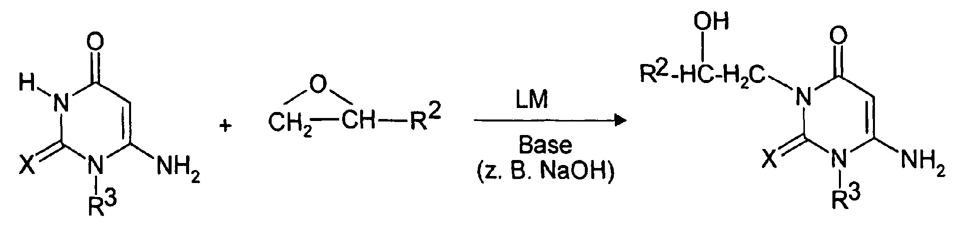 | | | | | | | |
|---|---|---|---|---|---|---|---|
| Beispiel | R² | R³ | X | LM | Smp. [°C] | Ausb. [%] | Bemerk. |
| 1 | -CH₂-OH | -CH₃ | O | H₂O | 198 - 201 | 60 | aus ⁿprOH |
| 2 | -CH₂-OH | -CH₂-CH₂-CH₃ | O | H₂O | 143 - 146 | 44 | aus ⁿPrOH/Aceton |
| 3 | -CH₂-OH | 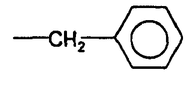 | O | H₂O/ⁿPrOH | 184 - 186 | 59 | aus ⁿPrOH enthält noch ca. 1 Mol ⁿPrOH |
| 4 | -CH₂-O-CH(CH₃)₂ | -CH₃ | O | H₂O/ⁿProH | 175 - 176 (trübe) | 60 | aus ⁿPrOH |
| 5 | -CH₂-O-CH(CH₃)₂ | 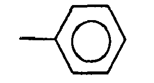 | O | H₂O/ⁿPrOH | 138 - 141 | 35 | aus Methyl^{t}butylether |
| 6 | -CH₂-O-C(CH₃)₃ | -CH₃ | O | H₂O/ⁿPrOH | 180 | 67 | aus ⁿPrOH |
| 7 | -CH₂-O-CH(CH₃)₂ | 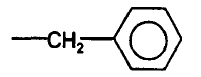 | O | H₂O/ⁿPrOH | 194 - 195 | 78 | aus ⁿPrOH |

| | | | | | | | |
|---|---|---|---|---|---|---|---|
| 8 | -CH₂-O-CO-neo-C₉H₁₉ | -CH₃ | O | H₂O/ⁿPrOH | 184 - 185 | 31 | aus MIBK |
| 9 | 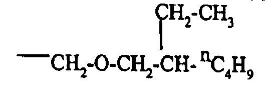 | -CH₃ | O | H₂O/ⁿPrOH | 161 - 162 | 23 | |
| 10 | -CH₃ | -CH₃ | O | H₂O/ⁿPrOH | 216 - 218 | 78 | aus H₂O/ⁿPrOH |
| 11 | -CH₃ | 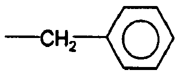 | O | H₂O/ⁿPrOH | 187 - 188 | 70 | aus H₂O/ⁿPrOH |
| 12 | -ⁿC₄H₉ | -CH₃ | O | H₂O/ⁿPrOH | 199 - 200 | 28 | aus ⁿPrOH |
| 13 | -CH₂-CH₃ | -CH₃ | O | H₂O/ⁿPrOH | 187 | 78 | aus H₂O/ⁿPrOH |
| 14 | -CH₂-CH₃ | 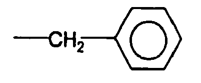 | O | H₂O/ⁿPrOH | 160 - 163 | 86 | aus ⁿPrOH |
| 15 | H | -CH₃ | O | H₂O | 284 - 85 | 84 | aus ⁿPrOH/H₂O |
| 16 | -CH₂-O-CH₂-CH=CH₂ | -CH₃ | O | H₂O | 150 - 152 | 84 | - |

**Tabelle 2**

| Reakt.: 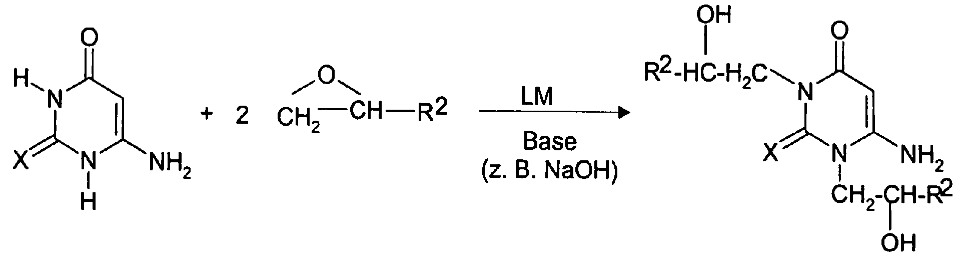 | | | | | | |
|---|---|---|---|---|---|---|
| Beispiel | R² | X | LM | Smp. [°C] | Ausb. [%] | Bemerk. |
| 17 | -CH₂-O-CH₂-CH=CH₂ | O | H₂O | - | 81 | nD30: 1,5225 |
| 18 | -CH₂-CH₃ | O | H₂O | - | - | gelbes Harz |
| 19 | H | O | H₂O | 170 - 171 | 41 | aus ⁿPrOH/H₂O |

### Beispiel I

### Statischer Hitzetest

Eine Trockenmischung bestehend aus

| | |
|---|---|
| 100,0 Teile | Evipol¹) SH 5730 = PVC K-Wert 57 |
| 5,0 Teile | Paraloid²) BTA III N 2 = MBS- (=Methylmethacrylat-Butadien-Styrol) Modifier |
| 0,5 Teile | Paraloid²) K 120 N = Acrylat Verarbeitungshilfe |
| 0,5 Teile | Paraloid²) K 175 = Acrylat Verarbeitungshilfe |
| 0,3 Teile | Wachs E = Esterwachs (Montan Wachs) (ex BASF) |
| 0,1 Teile | Loxiol® G 16 = Fettsäurepartialester des Glycerins (ex Henkel) |
| 3,0 Teile | ESO = epoxidiertes Sojabohnenöl |

| | |
|---|---|
| ¹⁾ Markenzeichen der Firma EVC ²⁾ Markenzeichen der Firma Rohm & Haas | |

und jeweils einer in der Tabelle 3 angegebenen Stabilisatoren wurde auf einem Mischwalzwerk 5 Minuten bei 180 °C gewalzt. Vom gebildeten Walzfell wurden Testfolienstreifen von 0,3 mm Dicke entnommen. Die Folienproben wurden in einem Ofen (=Mathis-Thermo-Takter) bei 190 °C thermisch belastet. Im zeitlichen Abstand von 3 Minuten wurde der Yellowness Index (YI) nach ASTM D-1925-70 bestimmt. Die Ergebnisse sind der folgenden Tabelle 3 zu entnehmen. Geringe YI- Werte bedeuten eine gute Stabilisierung.

**Tabelle 3**

| Minuten | ohne Stabilisator | Stabilisator 1 1,0 Teile | Stabilisator 2 1,0 Teile | Stabilisator 4 1,0 Teile | Stabilisator 7 1,0 Teile | Stabilisator 12 1,0 Teile |
|---|---|---|---|---|---|---|
| 0 | 68,10 | 10,82 | 6,99 | 6,35 | 7,57 | 7,17 |
| 3 | 75,63 | 13,37 | 9,63 | 7,28 | 8,94 | 7,89 |
| 6 | 99,65 | 17,15 | 12,31 | 8,64 | 10,93 | 8,97 |
| 9 | 130,67 | 23,47 | 16,06 | 11,78 | 14,71 | 11,68 |
| 12 | | 31,26 | 22,80 | 15,35 | 19,85 | 15,29 |
| 15 | | 40,88 | 29,16 | 19,91 | 26,10 | 20,81 |
| 18 | | 49,99 | 38,14 | 26,03 | 34,46 | 27,13 |
| 21 | | 61,17 | 48,49 | 32,42 | 45,56 | 35,59 |
| 24 | | 71,59 | 60,27 | 44,46 | 60,81 | 47,93 |
| 27 | | 87,03 | 74,30 | 63,65 | 76,53 | 68,02 |
| 30 | | 104,81 | 98,01 | 145,02 | 105,32 | 209,84 |

| | | | | | | |
|---|---|---|---|---|---|---|
| Legende Stabilisator 1 = Stabilisator entsprechend Synthesebeispiel 1 Stabilisator 2 = Stabilisator entsprechend Synthesebeispiel 2 Stabilisator 4 = Stabilisator entsprechend Synthesebeispiel 4 Stabilisator 7 = Stabilisator entsprechend Synthesebeispiel 7 Stabilisator 12 = Stabilisator entsprechend Synthesebeispiel 12 | | | | | | |

### Beispiel II

### Pressplatten

Eine Trockenmischung bestehend aus

| | |
|---|---|
| 100,0 Teile | Evipol¹) SH 5730 = PVC K-Wert 57 |
| 5,0 Teile | Paraloid²⁾ BTA III N 2 = MBS- (=Methylmethacrylat-Butadien-Styrol) Modifier |
| 0,5 Teile | Paraloid²⁾ K 120 N = Acrylat Verarbeitungshilfe |
| 0,5 Teile | Paraloid²⁾ K 175 = Acrylat Verarbeitungshilfe |
| 0,3 Teile | Wachs E = Esterwachs (Montan Wachs) (ex BASF) |
| 0,1 Teile | Loxiol® G 16 = Fettsäurepartialester des Glycerins (ex Henkel) |
| 3,0 Teile | ESO = epoxidiertes Sojabohnenöl |

| | |
|---|---|
| 1) Markenzeichen der Firma EVC 2) Markenzeichen der Firma Rohm & Haas | |

und jeweils einer in der Tabelle 4 angegebenen Stabilisatoren wurde auf einem Mischwalzwerk 5 Minuten bei 180 °C gewalzt. Die so gebildeten Walzfelle wurden bei 180 °C während einer Zeit von 1,5 min und einem Druck von 200 bar zu segmentierten Pressplatten mit einer Dicke von 1 mm verpresst. An diesen Pressplatten wurde der Yellowness Index (YI) nach ASTM D-1925-70 und die Transparenz nach ASTM D-2805-80 bzw. ASTM D-589-65 bestimmt. Die Ergebnisse sind der folgenden Tabelle 4 zu entnehmen. Geringe YI- Werte bedeuten eine gute Stabilisierung und hohe Transparenz-Werte bedeuten eine gute Transparenz der hergestellten Prüfkörper.

**Tabelle 4**

| Messdaten | ohne Stabilisator | Stabilisator 1 | Stabilisator 2 | Stabilisator 4 | Stabilisator 7 | Stabilisator 12 | Stabilisator 13 |
|---|---|---|---|---|---|---|---|
| YI | 190,1 | 34,7 | 19,9 | 14,5 | 16,0 | 11,7 | 9,2 |
| Transparenz (%) | 41,4 | 86,3 | 92,8 | 91,4 | 95,4 | 96,3 | 95,2 |

| | | | | | | | |
|---|---|---|---|---|---|---|---|
| Legende Stabilisator 1 = Stabilisator entsprechend Synthesebeispiel 1 Stabilisator 2 = Stabilisator entsprechend Synthesebeispiel 2 Stabilisator 4 = Stabilisator entsprechend Synthesebeispiel 4 Stabilisator 7 = Stabilisator entsprechend Synthesebeispiel 7 Stabilisator 12 = Stabilisator entsprechend Synthesebeispiel 12 Stabilisator 13 = Stabilisator entsprechend Synthesebeispiel 13 | | | | | | | |

## Patentansprüche

1. Verwendung einer Verbindung der allgemeinen Formel I 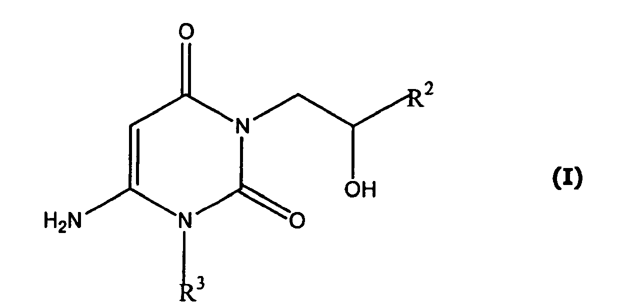 worin bedeuten,
R²= H, oder die Reste C₁-C₁₄-Alkyl, C₂-C₄-Alkenyl oder C₄C₈-Cycloalkyl, C₆-C₁₀-Aryl, Tolyl, Xylyl, Mesityl, Propylphenyl, Butylphenyl, Trimethoxyphenyl, Diethoxyphenyl, Propoxyphenyl und Butoxyphenyl, C₇-C₁₀-Aralkyl, -CH₂-O-R⁴, -CH₂-S-R⁴, mit
R⁴ = H, C₁-C₁₀-Alkylrest oder C₂-C₄-Alkenylrest oder
C₄-C₈-Cycloalkyl gegebenenfalls einen Oxiranring enthaltend oder gegebenenfalls substituiert mit 1-3 C₁-C₄-Alkyl, Benzoyl- oder C₂-C₁₈-Acylrest,
R³= R², R⁴ oder C₂-C₆-Alkyl mit mindestens 1 bis 5 OH-Gruppen substituiert und/oder durch mindestens 1 bis maximal 4 O-Atome unterbrochen oder C₃-C₈-Alkenyl oder -CH₂-CH(OH)R²
zur Stabilisierung chlorhaltiger Polymere.

2. Stabilisatorkombination enthaltend
A) 0,01 bis 10 Gew-Teile, bezogen auf 100 Gew-Teile chlorhaltigem Polymer, mindestens einer Verbindung der Formel I wie in Anspruch 1 angegeben und
B) eine oder mehrere Verbindungen aus der Gruppe der
B1) Polyole und Disaccharidalkohole,
B2) Perchlorat-Verbindungen
B3) Glycidylverbindungen, Epoxyverbindungen
B4) Hydrotalcite, Dawsonite
B5) Alkali- /Erdalkalialumosilikate (Zeolithe),
B6) Metallseifen
B7) Alkali- und Erdalkalioxide bzw. -hydroxide, -(hydrogen) carbonate oder- carboxylate
B8) Phosphite
B9) Antioxidantien, Lichtschutzmittel, Gleitmittel, Weichmacher,
B10) Pigmente, Füllstoffe.

3. Zusammensetzung, enthaltend ein chlorhaltiges Polymer und
A) 0,01 bis 10 Gew-Teile, bezogen auf 100 Gew-Teile chlorhaltigem Polymer, mindestens eine Verbindung der Formeln I wie in Anspruch 1 angegeben und gegebenenfalls B) eine oder mehrere Verbindungen aus der Gruppe der
B1) Polyole und Disaccharidalkohole,
B2) Perchlorat-Verbindungen
B3) Glycidylverbindungen, Epoxyverbindungen
B4) Hydrotalcite, Dawsonite
B5) Alkali- /Erdalkalialumosilikate (Zeolithe),
B6) Metallseifen
B7) Alkali- und Erdalkalioxide bzw. -hydroxide, -(hydrogen) carbonate oder- carboxylate
B8) Phosphite
B9) Antioxidantien, Lichtschutzmittel, Gleitmittel, Weichmacher,
B10) Pigmente, Füllstoffe.

## Claims

1. Use of a compound having the general formula I 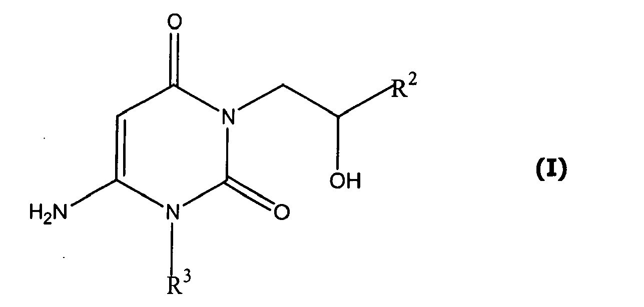 where R² = hydrogen or the radicals C₁-C₁₄ alkyl, C₂-C₄ alkenyl, or C₄-C₈ cycloalkyl, C₆-C₁₀ aryl, tolyl, xylyl, mesityl, propylphenyl, butylphenyl, trimethoxyphenyl, diethoxyphenyl, propoxyphenyl and butoxyphenyl, C₇-C₁₀ aralkyl, -CH₂-O-R⁴, -CH₂-S-R⁴, where
R⁴ = hydrogen, C₁-C₁₀ alkyl or C₂-C₄ alkenyl or C₄-C₈ cycloalkyl containing optionally an oxirane ring or substituted optionally with 1 to 3 C₁-C₄ alkyl, benzoyl or C₂-C₁₈ acyl groups,
R³= R², R⁴ or C₂-C₆ alkyl substituted with at least 1 to 5 OH groups and/or interrupted by at least 1 and not more than 4 oxygen atoms or C₃-C₈ alkenyl or -CH₂-CH(OH)R²
for stabilization of a chlorine-containing polymers.

2. A stabilizer combination containing
A) 0,01 to 10 parts by weight, based on 100 parts by weight of a chlorine-containing polymer, at least one compound of formula I as claimed in claim 1 and
B) one or more compounds selected from the group consisting of
B1) polyols and disaccharide alcohols,
B2) perchlorate compounds
B3) glycidyl compounds, epoxy compounds
B4) hydrotalcites, dawsonites
B5) alkali metal aluminosilicates, alkali earth metal aluminosilicates (zeolites),
B6) metal soaps
B7) alkali metal oxides and alkali earth metal oxides and alkali metal hydroxides, alkali metal (hydro)carbonate, alkali earth metal hydroxides, alkali earth metal (hydro)carbonate or alkali metal carboxylates, alkali earth metal carboxylates, respectively
B8) phosphites
B9) antioxidants, light stabilizers, lubricants, plasticizers,
B10) pigments, fillers.

3. A composition comprising a chlorine-containing polymer and
A) from 0,01 to 10 parts by weight, based on 100 parts by weight of chlorine-containing polymer, at least one compound of the formula I as claimed in claim 1 and optionally B) one or more compounds selected from the group consisting of
B1) polyols and disaccharide alcohols,
B2) perchlorate compounds
B3) glycidyl compounds, epoxy compounds
B4) hydrotalcites, dawsonites
B5) alkali metal aluminosilicates, alkali earth metal aluminosilicates (zeolites),
B6) metal soaps
B7) alkali metal oxides and alkali earth metal oxides and alkali metal hydroxides, alkali metal (hydro)carbonates, alkali earth metal hydroxides, alkali earth metal (hydro)carbonates or alkali metal carboxylates, alkali earth metal carboxylates, respectively
B8) phosphites
B9) antioxidants, light stabilizers, lubricants, plasticizers,
B10) pigments, fillers.

## Revendications

1. Utilisation d'un composé de formule générale I 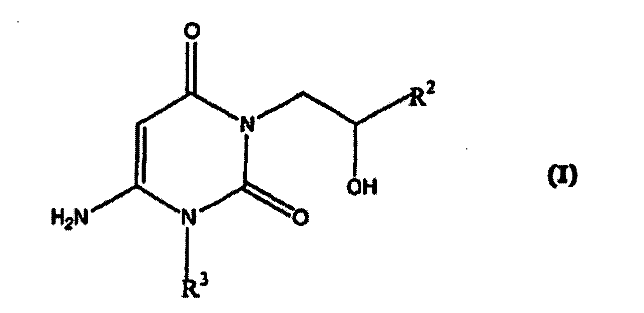 où
R² = H, ou les radicaux alkyle en C₁-C₁₄, alkényle en C₂-C₄, ou cycloalkyle en C₄-C₈, aryle en C₆-C₁₀, tolyle, xylyle, mésityle, propylphényle, butylphényle, triméthoxyphényle, diéthoxyphényle, propoxyphényle et butoxyphényle, aralkyle en C₇-C₁₀, -CH₂-O-R⁴ -CH₂-S-R⁴, avec
R⁴ = H, un radical alkyle en C₁-C₁₀ ou un radical alkényle en C₂-C₄ ou
un cycloalkyle en C₄-C₈ contenant éventuellement un cycle oxirane ou éventuellement substitué par 1 à 3 radicaux alkyle en C₁-C₄, benzoyle ou acyle en C₂-C₁₈,
R³ = R², R⁴ ou un alkyle en C₂-C₆ substitué par au moins 1 à 5 groupes OH et/ou interrompu par au moins 1 et jusqu'à 4 atomes O ou un alkényle en C₃-C₈ ou -CH₂-CH(OH)R²
pour stabiliser des polymères chlorés.

2. Combinaison de stabilisateurs contenant
A) 0,01 à 10 parties en poids, pour 100 parties en poids de polymère chloré, au moins un composé de formule I selon la revendication 1 et
B) un ou plusieurs composés issus du groupe des
B1) polyols et alcools disaccharidiques,
B2) composés perchloratés
B3) composés glycidyliques, composés époxydes
B4) hydrotalcites, dawsonites
B5) aluminosilicates alcalins et alcalinoterreux (zéolithes),
B6) savons métalliques
B7) oxydes ou hydroxydes, (hydrogéno) carbonates ou carboxylates alcalins et alcalinoterreux
B8) phosphites
B9) antioxydants, stabilisants à la lumière, lubrifiants, plastifiants,
B10) pigments, charges.

3. Composition contenant un polymère chloré et
A) 0,01 à 10 parties en poids, pour 100 parties en poids de polymère chloré, au moins un composé de formule I selon la revendication 1 et éventuellement B) un ou plusieurs composés issus du groupe des
B1) polyols et alcools disaccharidiques,
B2) composés perchloratés
B3) composés glycidyliques, composés époxydes
B4) hydrotalcites, dawsonites
B5) aluminosilicates alcalins et alcalinoterreux (zéolithes),
B6) savons métalliques
B7) oxydes ou hydroxydes, (hydrogéno) carbonates ou carboxylates alcalins et alcalinoterreux
B8) phosphites
B9) antioxydants, stabilisants à la lumière, lubrifiants, plastifiants,
B10) pigments, charges.
